# EUROPEAN PATENT APPLICATION

(11) **EP 3 327 659 A1**
(43) Date of publication of application: **30.05.2018**
(21) Application number: 17203011.6
(22) Date of filing: 22.11.2017
(51) Int. Cl.: G06Q 50/22

(54) **CLINICAL RESOURCE MANAGEMENT**

(30) Priority: 24.11.2016 CN 201611045155
(71) Applicant: Cardiac Pacemakers, Inc., St. Paul, MN 55112-5798 (US)
(72) Inventor: Zhang, Cheng, Ann Arbor, MI Michigan 48105 (US); Liu, Bei L., Huangpu District, Shanghai 20023 (CN); Cai, Shasha, PuDong District, Shanghai (CN); Xu, Feng K., Rizhao DongGang, 276800 (CN)
(74) Representative: Pfenning, Meinig & Partner mbB

(57) **Abstract**

A method of clinical resource management includes receiving an expert request associated with an expertise domain, where the expert request includes patient information, a proposed procedure date, an indication of a product, an indication of a procedure type, and/or an estimated cost. A caregiver, certified as an expert with regard to the expertise domain, is identified; and a notification of the expert request is provided to a caregiver device associated with the caregiver. The method includes receiving, from the caregiver device, an acceptance indication indicating that the caregiver has accepted the expert request; and providing, to the caregiver device associated with the selected caregiver, an acceptance confirmation indicating that the caregiver's acceptance satisfies the expert request.

## Description

### TECHNICAL FIELD

The present disclosure relates to systems and methods for facilitating management of clinical resources. More specifically, the disclosure relates to systems and methods for facilitating access to caregivers having certain expertise.

### BACKGROUND

In many countries such as, for example, China, experienced and well-trained caregivers typically are concentrated in the largest cities. There are many other cities that have significant populations but a shortage of caregivers having expertise with respect to certain expertise domains (e.g., products, procedures, etc.). The shortage of qualified caregivers may limit the spread of new medical technology, as patients in those cities would have to travel to bigger cities for various treatments, surgeries, medical devices, and/or the like. This uneven distribution of expertise is not only inconvenient but also may lead to dangerous situations. Additionally, for medical device providers, these regions may represent significant potential markets because there are large patient populations.

### SUMMARY

Embodiments include systems and methods that facilitate management of clinical resources.

In an Example 1, a method of clinical resource management, the method comprising: receiving an expert request associated with an expertise domain, the expert request comprising at least one of patient information, a proposed procedure date, an indication of a product, an indication of a procedure type, and an estimated cost; identifying a caregiver, wherein the caregiver is certified as an expert with regard to the expertise domain; providing, to a caregiver device associated with the caregiver, a notification of the expert request; receiving, from the caregiver device, an acceptance indication indicating that the caregiver has accepted the expert request; and providing, to the caregiver device associated with the selected caregiver, an acceptance confirmation indicating that the caregiver's acceptance satisfies the expert request.

In an Example 2, the method of Example 1, the expertise domain corresponding to at least one of a product and a procedure.

In an Example 3, the method of Example 1, further comprising: storing, in a caregiver record, caregiver information corresponding to the caregiver; receiving certification information associated with the caregiver, the certification information corresponding to an expertise domai2n; and certifying the caregiver as an expert with regard to the expertise domain.

In an Example 4, the method of Example 1, wherein certifying the caregiver as an expert with regard to the expertise domain, comprises: determining that the caregiver satisfies a set of expert criteria associated with the expertise domain; storing a certification indication that indicates that the caregiver is an expert with regard to the expertise domain; and associating the certification indication with the expertise domain and the caregiver record.

In an Example 5, the method of Example 1, further comprising receiving, from at least one additional caregiver device, at least one additional acceptance indication indicating that at least one additional caregiver has accepted the expert request.

In an Example 6, the method of Example 5, further comprising displaying, via an access device, a representation of the caregiver and at least one additional representation of the at least one additional caregiver.

In an Example 7, the method of Example 6, further comprising receiving, from the access device, an indication of a user selection of a selected caregiver, the selected caregiver comprising one of the caregiver and the at least one additional caregiver.

In an Example 8, the method of any of Examples 1-7, further comprising: creating, via a scheduling platform, an appointment corresponding to the expert request; and providing, to the caregiver device associated with the selected caregiver, an appointment confirmation associated with the appointment.

In an Example 9, the method of any of Examples 1-8, further comprising: receiving an evaluation of the selected caregiver, the evaluation comprising evaluation information; and associating, with the caregiver record, at least one of the evaluation information and information derived from the evaluation information, the information derived from the evaluation information comprising a quality score.

In an Example 10, a health management system, the system comprising: at least one processor; and one or more computer-readable media having computer-executable instructions embodied thereon that, when executed by the at least one processor, cause the at least one processor to instantiate at least one program component, the at least one program component comprising a medical link manager configured to: receive an expert request associated with an expertise domain, the expertise domain corresponding to at least one of a product and a procedure, the expert request comprising at least one of patient information, a proposed procedure date, an indication of a product, an indication of a procedure type, and an estimated cost; identify a caregiver, wherein the caregiver is certified as an expert with regard to the expertise domain; provide, to a caregiver device associated with the caregiver, a notification of the expert request; receive, from the caregiver device, an acceptance indication indicating that the caregiver has accepted the expert request; and provide, to the caregiver device associated with the selected caregiver, an acceptance confirmation indicating that the caregiver's acceptance satisfies the expert request.

In an Example 11, the system of Example 10, the at least one program component further comprising a registration/certification component configured to: store, in a caregiver record, caregiver information corresponding to the caregiver; receive certification information associated with the caregiver, the certification information corresponding to an expertise domain; and certify the caregiver as an expert with regard to the expertise domain, wherein the registration/certification component is configured to certify the caregiver as an expert with regard to the expertise domain by: determining that the caregiver satisfies a set of expert criteria associated with the expertise domain; storing a certification indication that indicates that the caregiver is an expert with regard to the expertise domain; and associating the certification indication with the expertise domain and the caregiver record.

In an Example 12, the system of either of Examples 10 or 11, wherein the medical link manager is further configured to: receive, from at least one additional caregiver device, at least one additional acceptance indication indicating that at least one additional caregiver has accepted the expert request; and receive an indication of a user selection of a selected caregiver, the selected caregiver comprising one of the caregiver and the at least one additional caregiver.

In an Example 13, the system of any of Examples 10-12, further comprising a scheduling platform configured to: create an appointment corresponding to the expert request; and provide, to the caregiver device associated with the selected caregiver, an appointment confirmation associated with the appointment.

In an Example 14, the system of any of Examples 10-12, further comprising a feedback component configured to: provide, to at least one of a facility device and a patient device, an evaluation user interface, the evaluation user interface comprising input mechanisms for receiving evaluation information; receive, via the evaluation user interface, an evaluation of the selected caregiver, the evaluation comprising evaluation information; and associate, with the caregiver record, at least one of the evaluation information and information derived from the evaluation information, the information derived from the evaluation information comprising a quality score.

In an Example 15, a method of clinical resource management, the method comprising: creating, in a database stored in computer memory, a first caregiver record and a second caregiver record, the first and second caregiver records corresponding to a first caregiver and a second caregiver, respectively; receiving a first set of certification information and a second set of certification information, the first set of certification information and the second set of certification information corresponding to the first caregiver and the second caregiver, respectively; determining, based on the first set of certification information, that the first caregiver satisfies a first set of expert criteria associated with an expertise domain, the expertise domain corresponding to at least one of a product and a procedure; storing a first certification indication, the first certification indication indicating that the first caregiver is an expert with regard to the expertise domain; associating the first certification indication with the expertise domain and the first caregiver record; determining, based on the second set of certification information, that the second caregiver satisfies a set of expert criteria associated with the expertise domain; storing a second certification indication, the second certification indication indicating that the second caregiver is an expert with regard to the expertise domain; associating the second certification indication with the expertise domain and the second caregiver record; receiving an expert request associated with the expertise domain; identifying, based on the first and second certification indications, the first and second caregivers, respectively; providing, to a first caregiver device associated with the first caregiver, a first notification of the expert request; providing, to a second caregiver device associated with the second caregiver, a second notification of the expert request; receiving, from the first and second caregiver devices, a first acceptance indication and a second acceptance indication, respectively, the first and second acceptance indications respectively indicating that the first and second caregivers have accepted the expert request; providing, to an access device, a representation of each of the first and second caregivers; receiving, from the access device, an indication of a selection of a selected caregiver, the selected caregiver comprising the first caregiver; and providing, to the first caregiver device, an acceptance confirmation indicating that the caregiver's acceptance satisfies the expert request.

In an Example 16, a method of clinical resource management, the method comprising: receiving an expert request associated with an expertise domain, the expert request comprising at least one of patient information, a proposed procedure date, an indication of a product, an indication of a procedure type, and an estimated cost; identifying a caregiver, wherein the caregiver is certified as an expert with regard to the expertise domain; providing, to a caregiver device associated with the caregiver, a notification of the expert request; receiving, from the caregiver device, an acceptance indication indicating that the caregiver has accepted the expert request; and providing, to the caregiver device associated with the selected caregiver, an acceptance confirmation indicating that the caregiver's acceptance satisfies the expert request.

In an Example 17, the method of Example 16, the expertise domain corresponding to at least one of a product and a procedure.

In an Example 18, the method of Example 16, further comprising: storing, in a caregiver record, caregiver information corresponding to the caregiver; receiving certification information associated with the caregiver, the certification information corresponding to an expertise domain; and certifying the caregiver as an expert with regard to the expertise domain.

In an Example 19, the method of Example 16, wherein certifying the caregiver as an expert with regard to the expertise domain, comprises: determining that the caregiver satisfies a set of expert criteria associated with the expertise domain; storing a certification indication that indicates that the caregiver is an expert with regard to the expertise domain; and associating the certification indication with the expertise domain and the caregiver record.

In an Example 20, the method of Example 16, further comprising receiving, from at least one additional caregiver device, at least one additional acceptance indication indicating that at least one additional caregiver has accepted the expert request.

In an Example 21, the method of Example 20, further comprising: displaying, via a user interface, a representation of the caregiver and at least one additional representation of the at least one additional caregiver; and receiving, via the user interface, an indication of a user selection of a selected caregiver, the selected caregiver comprising one of the caregiver and the at least one additional caregiver.

In an Example 22, the method of Example 20, further comprising automatically selecting a caregiver, the selected caregiver comprising one of the caregiver and the at least one additional caregiver.

In an Example 23, the method of Example 16, further comprising: creating, via a scheduling platform, an appointment corresponding to the expert request; and providing, to the caregiver device associated with the selected caregiver, an appointment confirmation associated with the appointment.

In an Example 24, the method of Example 16, further comprising: receiving, from at least one of the facility device and a patient device, an evaluation of the selected caregiver, the evaluation comprising evaluation information; and associating, with the caregiver record, at least one of the evaluation information and information derived from the evaluation information, the information derived from the evaluation information comprising a quality score.

In an Example 25, a health management system, the system comprising: at least one processor; and one or more computer-readable media having computer-executable instructions embodied thereon that, when executed by the at least one processor, cause the at least one processor to instantiate at least one program component, the at least one program component comprising a medical link manager configured to: receive an expert request associated with an expertise domain, the expert request comprising at least one of patient information, a proposed procedure date, an indication of a product, an indication of a procedure type, and an estimated cost; identify a caregiver, wherein the caregiver is certified as an expert with regard to the expertise domain; provide, to a caregiver device associated with the caregiver, a notification of the expert request; receive, from the caregiver device, an acceptance indication indicating that the caregiver has accepted the expert request; and provide, to the caregiver device associated with the selected caregiver, an acceptance confirmation indicating that the caregiver's acceptance satisfies the expert request.

In an Example 26, the system of Example 25, the expertise domain corresponding to at least one of a product and a procedure.

In an Example 27, the system of Example 25, the at least one program component further comprising a registration/certification component configured to: store, in a caregiver record, caregiver information corresponding to the caregiver; receive certification information associated with the caregiver, the certification information corresponding to an expertise domain; and certify the caregiver as an expert with regard to the expertise domain, wherein certifying the caregiver as an expert with regard to the expertise domain, comprises: determining that the caregiver satisfies a set of expert criteria associated with the expertise domain; storing a certification indication that indicates that the caregiver is an expert with regard to the expertise domain; and associating the certification indication with the expertise domain and the caregiver record.

In an Example 28, the system of Example 25, wherein the medical link manager is further configured to: receive, from at least one additional caregiver device, at least one additional acceptance indication indicating that at least one additional caregiver has accepted the expert request; and receive an indication of a user selection of a selected caregiver, the selected caregiver comprising one of the caregiver and the at least one additional caregiver.

In an Example 29, the system of Example 25, further comprising a scheduling platform configured to: create an appointment corresponding to the expert request; and provide, to the caregiver device associated with the selected caregiver, an appointment confirmation associated with the appointment.

In an Example 30, the system of Example 25, further comprising a risk analyzer configured to: receive a set of patient information associated with a patient; determine, based on the set of patient information, a risk score indicating a likelihood that the patient may benefit from a treatment corresponding to the expertise domain; determine that the risk score exceeds a specified threshold; and generate, in response to determining that the risk score exceeds the specified threshold, the expert request.

In an Example 31, a method of clinical resource management, the method comprising: creating, in a database stored in computer memory, a first caregiver record and a second caregiver record, the first and second caregiver records corresponding to a first caregiver and a second caregiver, respectively; receiving a first set of certification information and a second set of certification information, the first set of certification information and the second set of certification information corresponding to the first caregiver and the second caregiver, respectively; determining, based on the first set of certification information, that the first caregiver satisfies a first set of expert criteria associated with an expertise domain, the expertise domain corresponding to at least one of a product and a procedure; storing a first certification indication, the first certification indication indicating that the first caregiver is an expert with regard to the expertise domain; associating the first certification indication with the expertise domain and the first caregiver record; determining, based on the second set of certification information, that the second caregiver satisfies a set of expert criteria associated with the expertise domain; storing a second certification indication, the second certification indication indicating that the second caregiver is an expert with regard to the expertise domain; associating the second certification indication with the expertise domain and the second caregiver record; receiving an expert request associated with the expertise domain; identifying, based on the first and second certification indications, the first and second caregivers, respectively; providing, to a first caregiver device associated with the first caregiver, a first notification of the expert request; providing, to a second caregiver device associated with the second caregiver, a second notification of the expert request; receiving, from the first and second caregiver devices, a first acceptance indication and a second acceptance indication, respectively, the first and second acceptance indications respectively indicating that the first and second caregivers have accepted the expert request; providing, to at least one of a facility device and a patient device, caregiver information associated with the first and second caregivers; receiving, from the facility device, an indication of a selection of a selected caregiver, the selected caregiver comprising the first caregiver; and providing, to the first caregiver device, an acceptance confirmation indicating that the caregiver's acceptance satisfies the expert request.

In an Example 32, the method of Example 31, further comprising: creating, via a scheduling platform, an appointment corresponding to the expert request; and providing, to the caregiver device associated with the selected caregiver, an appointment confirmation associated with the appointment.

In an Example 33, the method of Example 31, further comprising: receiving, from at least one of the facility device and a patient device, an evaluation of the selected caregiver, the evaluation comprising evaluation information; and associating, with the first caregiver record, at least one of the evaluation information and information derived from the evaluation information, the information derived from the evaluation information comprising a quality score.

In an Example 34, the method of Example 33, wherein the caregiver information associated with the first caregiver includes a prior quality score.

In an Example 35, the method of Example 31, wherein determining that the first caregiver satisfies a first set of expert criteria associated with an expertise domain comprises determining that the first caregiver has completed a specified level of training associated with the expertise domain.

While multiple embodiments are disclosed, still other embodiments of the present disclosure will become apparent to those skilled in the art from the following detailed description, which shows and describes illustrative embodiments of the disclosure. Accordingly, the drawings and detailed description are to be regarded as illustrative in nature and not restrictive.

### BRIEF DESCRIPTION OF THE DRAWINGS

FIG. 1 is a block diagram depicting an illustrative system 100 for facilitating management of clinical resources, in accordance with embodiments of the disclosure.
FIG. 2 is a block diagram depicting an illustrative computing device 200, in accordance with embodiments of the disclosure.
FIG. 3 is a block diagram depicting an illustrative system 300 for facilitating management of clinical resources, in accordance with embodiments of the disclosure.
FIG. 4 is a flow diagram depicting an illustrative method of facilitating management of clinical resources, in accordance with embodiments of the disclosure.
FIG. 5 is a flow diagram depicting another illustrative method of facilitating management of clinical resources, in accordance with embodiments of the disclosure.

While the disclosed subject matter is amenable to various modifications and alternative forms, specific embodiments have been shown by way of example in the drawings and are described in detail below. The intention, however, is not to limit the disclosed subject matter to the particular embodiments described. On the contrary, the disclosure is intended to cover all modifications, equivalents, and alternatives falling within the scope of the disclosed subject matter as defined by the appended claims.

As the terms are used herein with respect to ranges of measurements (such as those disclosed immediately above), "about" and "approximately" may be used, interchangeably, to refer to a measurement that includes the stated measurement and that also includes any measurements that are reasonably close to the stated measurement, but that may differ by a reasonably small amount such as will be understood, and readily ascertained, by individuals having ordinary skill in the relevant arts to be attributable to measurement error, differences in measurement and/or manufacturing equipment calibration, human error in reading and/or setting measurements, adjustments made to optimize performance and/or structural parameters in view of differences in measurements associated with other components, particular implementation scenarios, imprecise adjustment and/or manipulation of objects by a person or machine, and/or the like.

Although the term "block" may be used herein to connote different elements illustratively employed, the term should not be interpreted as implying any requirement of, or particular order among or between, various blocks disclosed herein. Similarly, although illustrative methods may be represented by one or more drawings (e.g., flow diagrams, communication flows, etc.), the drawings should not be interpreted as implying any requirement of, or particular order among or between, various steps disclosed herein. Additionally, a "set," "subset," or "group" of items (e.g., inputs, algorithms, data values, etc.) may include one or more items, and, similarly, a subset or subgroup of items may include one or more items. A "plurality" means more than one.

### DETAILED DESCRIPTION

Embodiments include health management systems and methods that facilitate patient health management, prevention of patient health deterioration, prevention of patient adverse events, patient care planning and execution, clinical resource management, and/or the like. Embodiments include a health management system configured to manage clinical resources, which may include, for example, linking caregivers certified as experts with respect to certain expertise domains with facilities that could benefit from the service of those experts. Conventional clinical resource management systems typically manage resources of a single facility or network of facilities based on internal inventory and prioritization information, but those systems generally are not configured to identify experts not otherwise associated with the facility, facilitate communicating expert requests to such experts, and/or the like. Embodiments of the health management system described herein incorporate these expert identification and linking strategies, which may facilitate more flexible and robust management of clinical resources.

Embodiments of the health management system incorporate access to different databases or repositories containing different types of information, thereby facilitating increasingly sophisticated caregiver selection that takes into account numerous aspects of a caregiver's expertise, experience, and performance, as well as the patient's condition, situation, preferences, values, culture, behaviors, and/or the like. For example, the health management system may receive information from one or more information sources that provide a patient's clinical information such as, for example, an electronic health record (EHR) system, and/or a personal health record (PHR) system. The system also may receive information from a Patient Relationship Management (PRM) system, which provides other types of information that may facilitate understanding an individual patient's risks and various factors that contribute to those risks. For example, the PRM system may provide psychosocial information, experiential information, relational information, preferential information, demographic information, cultural information, and/or the like. Embodiments of the PRM system may be used for documenting, planning and facilitating patient care episodes and/or patient interactions. For example, the PRM system (which may, in embodiments, be integrated with the health management system) may provide a PRM dashboard configured to present patient information, information about past interactions, previous efforts to follow-up on or reach out to a patient, and/or the like.

Embodiments of the health management system may facilitate user access to information from systems such as the EHR, PHR, and PRM systems by providing interfaces to those systems, by providing a query service that access those systems, by integrating those systems within the health management system, and/or the like. Embodiments of the health management system may implement guidelines and/or algorithms that enable it to provide healthcare providers with recommendations and/or prompts (health planning recommendations) to facilitate assembly of an appropriate patient care team and care plan based on risk scores calculated and risk factors identified, expertise domains implicated, and/or the like. Embodiments may facilitate prioritization of treatment for patients and/or symptoms, distribution of clinical resources, and/or the like, thereby enabling workflow efficiencies.

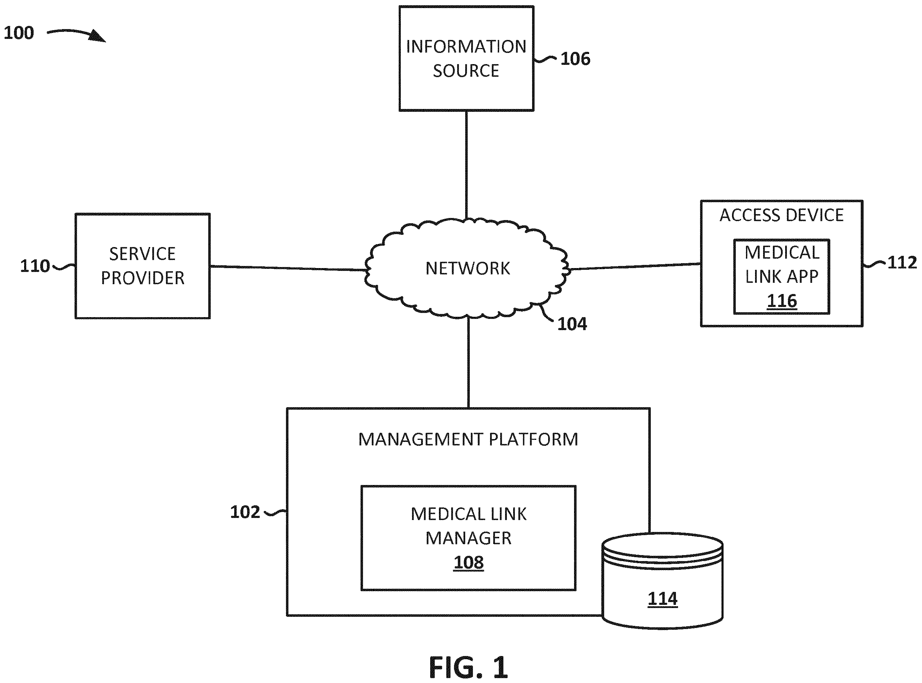

FIG. 1 is a block diagram depicting an illustrative system 100 for facilitating providing health care, in accordance with embodiments of the disclosure. As shown in FIG. 1, the illustrative health management system 100 includes a management platform 102 that accesses patient information, via a network 104, from an information source 106. The network 104 may be, or include, any number of different types of communication networks such as, for example, a short messaging service (SMS), a local area network (LAN), a wireless LAN (WLAN), a virtual LAN (VLAN), a wide area network (WAN), the Internet, a peer-to-peer (P2P) network, custom-designed communication or messaging protocols, and/or the like. The network 104 may include a combination of multiple networks. The information source 106 may include, for example, the Internet, an email provider, a website, an information service, an electronic health record (EHR), a patient relationship management (PRM) database, a user interface, and/or the like.

According to embodiments, the management platform 102 implements a medical link manager 108 that facilitates managing clinical resources such as, for example, by facilitating identification of experts with regard to particular expert domains and facilitating communications with the identified experts to facilitate scheduling appointments with the experts. The medical link manager 108 may use the accessed information to determine any number of different care metrics which may, in embodiments, facilitate identification and/or selection of experts associated with an expertise domain. The management platform 102 may facilitate any number of health-management related services such as, for example, by providing access to the care metrics, caregiver information, and related information, and/or by utilizing a service provider 110, which a consumer of the services may access with an access device 112. Although depicted as a single component solely for the purposes of clarity of description, the access device 112 may actually refer to more than one access device 112.

The management platform 102, the information source 106, and/or the service provider 110 may be implemented using one or more servers, which may be, include, or may be included in, a computing device that includes one or more processors and a memory. The one or more servers, and/or any one or more components thereof, may be implemented in a single server instance, multiple server instances (e.g., as a server cluster), distributed across multiple computing devices, instantiated within multiple virtual machines, implemented using virtualized components such as virtualized processors and memory, and/or the like. According to embodiments, the management platform 102 may be referred to as a care management platform or care coordination platform.

The medical link manager 108 obtains, copies, or otherwise accesses patient information from the information source 106. Although depicted as a single component solely for the purposes of clarity of description, the information source 106 may actually refer to more than one information source 106. The medical link manager 108 may store the patient information, caregiver information, product information, procedure information, and/or market information; portions of the patient information, caregiver information, product information, procedure information, and/or market information; and/or information extracted from the patient information, caregiver information, product information, procedure information, and/or market information in a database 114. The database 114, which may refer to one or more databases, may be, or include, one or more tables, one or more relational databases, one or more multidimensional data cubes, and the like. Further, though illustrated as a single component, the database 114 may, in fact, be a plurality of databases 114 such as, for instance, a database cluster, which may be implemented on a single computing device or distributed between a number of computing devices, memory components, or the like.

In operation, the medical link manager 108 may be configured to receive an expert request associated with an expertise domain and identify a caregiver that is certified as an expert with regard to the expertise domain. The expert request may include patient information, a proposed procedure date, an indication of a product, an indication of a procedure type, an estimated cost, and/or the like. The medical link manager 108 may also be configured to provide, to a caregiver device associated with the caregiver, a notification of the expert request; and to receive, from the caregiver device, an acceptance indication indicating that the caregiver has accepted the expert request.

In embodiments, more than one caregiver may accept an expert request, or a caregiver that accepts an expert request may be otherwise unqualified to satisfy the expert request. Thus, the medical link manager 108 may be further configured to provide, to the caregiver device associated with the selected caregiver, an acceptance confirmation indicating that the caregiver's acceptance satisfies the expert request. An acceptance confirmation may be generated in response, for example, to receiving a selection of the caregiver by a facility representative, a patient, or an automated (or semi-automated) process.

For example, in embodiments, the medical link manager 108 may be configured to receive, from a caregiver device, an acceptance indication indicating that the associated caregiver has accepted the expert request, and to receive, from at least one additional caregiver device, at least one additional acceptance indication indicating that at least one additional caregiver has accepted the expert request. The medical link manager 108 may be configured to provide caregiver information, associated with the caregiver and the at least one additional caregiver, to a facility administrator, another caregiver (e.g., a clinician associated with the facility), the patient associated with the expert request, and/or the like. In this manner, the medical link manager 108 may facilitate selection of a caregiver to satisfy the expert request.

For example, the medical link manager 108 may provide a representation of each of the caregivers that accepted the expert request to a medical link application instantiated by a facility device (e.g., a facility application) and/or a medical link application instantiated by a patient device (e.g., a patient application). The facility application and/or the patient application may be configured to display the representations of the caregivers. The representations may, in embodiments, include selectable options such that, upon selection thereof by a user, the facility application and/or patient application may be configured to display caregiver information. In embodiments, the facility application and/or patient application may be configured to request, from the medical link manager 108, caregiver information associated with a particular caregiver in response to receiving a user selection of a selectable option corresponding to that caregiver. In embodiments, the caregiver information may include any number of different types of information associated with a caregiver such as, for example, the caregiver's name, the caregiver's occupation, the caregiver's title, the caregiver's residence (e.g., city, state, region, etc.), an indication of each expertise domain with respect to which the caregiver is certified as an expert, information about the caregiver's experience, one or more evaluation scores (e.g., scores calculated based on evaluation information received from a patient, caregiver, and/or the like regarding the performance, service, and/or abilities of the caregiver - e.g., in reference to a prior procedure), evaluation information, and/or the like.

The patient and/or facility caregiver may use the caregiver information to inform a selection of a caregiver to satisfy the expert request. According to embodiments, the facility application and/or patient application may be configured to present a selectable option for selecting a caregiver. Upon receiving a user selection of the selectable option for selecting a caregiver, the facility application and/or patient application may be configured to provide an indication of that selection to the medical link manager 108. In turn, the medical link manager 108 may be configured to receive, from the facility application and/or the patient application, an indication of a selection of the selected caregiver. In response to receiving this indication, the medical link manager 108 may be configured to provide, to the caregiver device associated with the selected caregiver, an acceptance confirmation indicating that the caregiver's acceptance satisfies the expert request.

According to embodiments, the medical link manager 108 may be configured to use an automated process to select a caregiver to satisfy the expert request, to suggest a selection of a caregiver, and/or the like. The medical link manager 108 may be configured to evaluate any number of different types of information as part of the selection and/or suggestion process such as, for example, patient information, caregiver information, evaluation information, clinician information, product information, market information, and/or the like. For example, in embodiments, the medical link manager 108 is configured to access patient information, caregiver information, product information, procedure information, and/or market information (e.g., from the database 114, the information source 106, and/or the like) and, based on the expert request, patient information, caregiver information, product information, procedure information, and/or market information, determine one or more caregiver metrics. The medical link manager 108 may be further configured to, based on the one or more caregiver metrics, select a caregiver, suggest a selection of a caregiver, and/or the like. As used herein, the term "based on" is not meant to be restrictive, but rather indicates that a determination, identification, prediction, calculation, or the like, is performed by using, at least, the term following "based on" as an input. For example, a medical link manager 108 that determines a caregiver metric based on a particular piece of information may additionally, or alternatively, base the same determination on another piece of information.

In embodiments, the medical link manager 108 may include a neural network, fuzzy logic system, or similar system for analyzing information to facilitate the selection and/or suggestion of caregivers. Further, the medical link manager 108 may also provide means for periodic processing of present and historical data to yield a multidimensional caregiver performance indication along with outcome trend prediction. The medical link manager 108 may also integrate data collected from internal and external devices with subjective data to optimize management of overall patient health.

The medical link manager 108 may also perform any number of deterministic and/or probabilistic calculations. For example, the medical link manager 108 may be configured to gather data related to a caregiver's performance (e.g., as characterized using evaluation information provided by patients and/or caregivers), and provide suggestions and/or selections based on this analysis. In embodiments, the management platform 102 may be used as a "data clearinghouse," to gather and integrate data collected from medical devices and/or other sources (such as, for example, the information source 106, the service provider 110, and/or the access device 112), as well as information from sources outside the health management system 100. The integrated information may be shared with other interested entities, subject to privacy restrictions, thereby increasing the quality and integration of data available.

In embodiments, the medical link manager 108 determines a caregiver metric based on a number of different types of caregiver information. In addition to using EHR information and PRM information, embodiments of the medical link manager 108 may use various user inputs in determining a caregiver metric. For example, a user (e.g., a patient, a caregiver, an insurer, etc.) may want to obtain a caregiver metric associated with a certain caregiver, and may do so by providing a query to the health management system 100 as an input. Caregiver metrics may include any number of different types of information resulting from analyses of caregiver information, patient information, and/or the like.

In embodiments, the caregiver metrics, expert requests, and/or the like, may be used to facilitate one or more services. Aspects of the services may be provided using the management platform 102 and/or the service provider 110 which may include, for example, applications, service functions, or the like, that provide services for facilitating management of clinical resources. In embodiments, the service provider 110 may refer to one or more service providers 110 any one or more of which (and/or components thereof) may be integrated with the management platform 102. In embodiments, the services may include presenting caregiver information to users; identifying experts with respect to expertise domains; facilitating communication with caregivers; providing caregiver recommendations and/or selections; identifying appropriate members of a care team; providing notifications of certain events and/or care episodes to caregivers; certifying caregivers as experts with respect to expertise domains; promoting and/or tracking the use of certain products; providing financial information associated with expertise domains; evaluating caregiver performance; and/or the like.

Embodiments of the management platform 102 and/or the service provider 110 may be configured to provide clinical decision support or recommendations for changes to existing practices. For example, in embodiments, a service may include providing a recommendation to a provider about what next care pathway steps should be implemented for a patient, which expertise domains are implicated in a patient's care, and/or the like. In embodiments, the management platform 102 and/or the service provider 110 may be used to evaluate the effectiveness of treatment and may be used for pilot studies or clinical trials.

Various components depicted in FIG. 1 may operate together to form the health management system 100, which may be, for example, a computerized patient management and monitoring system. In embodiments, the system 100 may be designed to assist in monitoring the patient's condition, managing the patient's therapy, and/or the like. Patient health management and monitoring systems can provide large amounts of data about patients to users such as, for example, clinicians, patients, researchers, and/or the like. According to embodiments, the management platform 102 may additionally, or alternatively, be configured to provide reports to access devices 112, manage patient information, configure therapy regimens, manage/update device software, and/or the like.

The management platform 102 may be configured to perform security functions, verification functions, and/or the like. Due to potential risks associated with inaccurate calculation of risk scores and recommendations generated based thereon, it may be desirable for aspects of an at least partially automated system 100 to include safeguards such as, for example, verification of calculations, clinician oversight, and/or the like. For example, some types of users may be permitted, by the management platform 102, to have access to different amounts and/or types of information than other users. In embodiments, the management platform 102 may facilitate maintaining user profiles so that a user's role can be verified, thereby enabling the management platform 102 to customize the information available to a user. That is, for instance, an insurer may only be permitted to access certain portions of an EHR, PHR, PRM database, risk score, and/or the like, whereas a member of a patient's care team may be permitted to access more information.

In embodiments, the health management system 100 may be configured so that various components of the health management system 100 provide reporting to various individuals (e.g., patients and/or caregivers). For example, in embodiments, a user interface can be accessed via a device that is portable such that the user can use the system and have access to the system as they move about within a hospital. In addition to forms of reporting including visual and/or audible information, the system 100 may also communicate with and/or reconfigure medical devices, which may be examples of information sources 106 and/or access devices 112. For example, if an access device 112 is part of a cardiac rhythm management system, the management platform 102 may communicate with the device 112 and reconfigure the therapy provided by the cardiac rhythm management system based on the patient information. In another embodiment, the management platform 102 may provide, to the access device 112, recorded information, an ideal range for the information, a conclusion based on the information, a recommended course of action, and/or the like. This information may be displayed using a display device associated with the access device 112 for the patient to review or made available for the patient and/or clinician to review.

A variety of communication methods and protocols may be used to facilitate communication between management platforms 102, information sources 106, service providers 110, and/or access devices 112. For example, wired and wireless communications methods may be used. Wired communication methods may include, for example and without limitation, traditional copper-line communications such as DSL, broadband technologies such as ISDN and cable modems, and fiber optics, while wireless communications may include cellular, satellite, radio frequency (RF), Infrared, and/or the like.

For any given communication method, a multitude of standard and/or proprietary communication protocols may be used. For example and without limitation, protocols such as radio frequency pulse coding, spread spectrum, direct sequence, time-hopping, frequency hopping, SMTP, FTP, and TCP/IP may be used. Other proprietary methods and protocols may also be used. Further, a combination of two or more of the communication methods and protocols may also be used.

The various communications between the components of the system 100 may be made secure using several different techniques. For example, encryption and/or tunneling techniques may be used to protect data transmissions. Alternatively, a priority data exchange format and interface that are kept confidential may also be used. Authentication may be implemented using, for example, digital signatures based on a known key structure (e.g., PGP or RSA). Other physical security and authentication measures may also be used, such as security cards and biometric security apparatuses (e.g., retina scans, iris scans, fingerprint scans, vein-print scans, voice, facial geometry recognition, etc.). Conventional security methods such as firewalls may be used to protect information residing on one or more of the storage media of the advanced patient management system 100. Encryption, authentication and verification techniques may also be used to detect and correct data transmission errors.

In embodiments, varying levels of security may be applied to communications depending on the type of information being transmitted. For example, in embodiments, the management platform 102 (or other device) may be configured to apply stricter security measures to confidential health care information than to demographic information. Similarly, even more security may be applied to communications of information used for controlling therapy, adjudicating episodes, and/or the like. In embodiments, varying levels of security may be applied to communications depending on the type of user to whom the information is being communicated. Additionally, in embodiments, communications among the various components of the system 100 may be enhanced using compression techniques to allow large amounts of data to be transmitted efficiently.

The illustrative health management system 100 shown in FIG. 1 is not intended to suggest any limitation as to the scope of use or functionality of embodiments of the present disclosure. Neither should the illustrative system 100 be interpreted as having any dependency or requirement related to any single component or combination of components illustrated therein. Additionally, various components depicted in FIG. 1 may be, in embodiments, integrated with various ones of the other components depicted therein (and/or components not illustrated), all of which are considered to be within the ambit of the present disclosure.

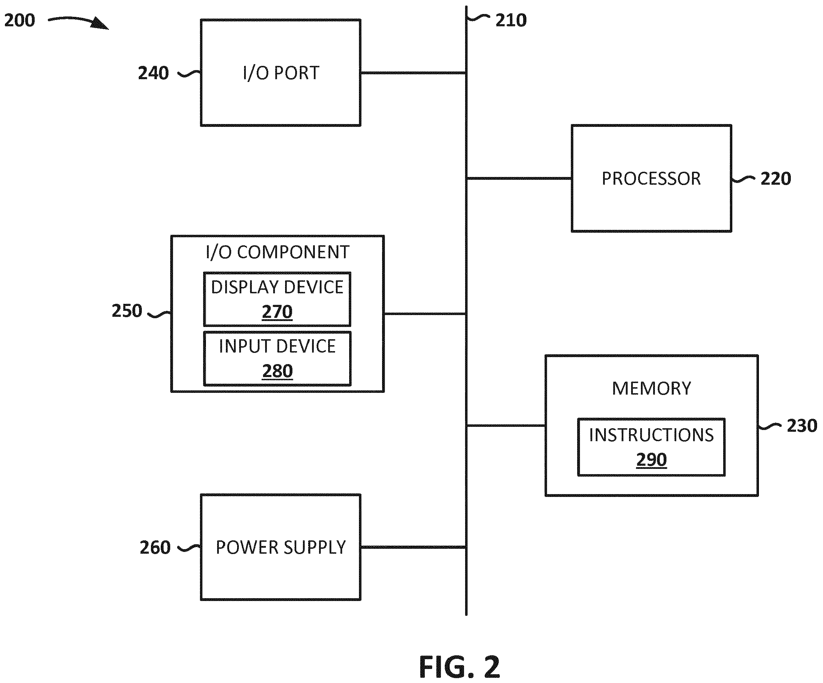

According to various embodiments of the disclosed subject matter, any number of the components depicted in FIG.1 (e.g., the management platform 102, the information source 106, the service provider 110, and/or the access device 112) may be implemented on one or more computing devices. FIG. 2 is a block diagram depicting an illustrative computing device 200, in accordance with embodiments of the disclosure. The computing device 200 may include any type of computing device suitable for implementing aspects of embodiments of the disclosed subject matter. Examples of computing devices include specialized computing devices or general-purpose computing devices such "workstations," "servers," "laptops," "desktops," "tablet computers," "hand-held devices," "general-purpose graphics processing units (GPGPUs)," and the like, all of which are contemplated within the scope of FIGS. 1 and 2, with reference to various components of the system 100 and/or computing device 200.

In embodiments, the computing device 200 includes a bus 210 that, directly and/or indirectly, couples the following devices: a processor 220, a memory 230, an input/output (I/O) port 240, an I/O component 250, and a power supply 260. Any number of additional components, different components, and/or combinations of components may also be included in the computing device 200. The I/O component 250 may include a presentation component configured to present information to a user such as, for example, a display device 270, a speaker, a printing device, and/or the like, and/or an input device 280 such as, for example, a microphone, a joystick, a satellite dish, a scanner, a printer, a wireless device, a keyboard, a pen, a voice input device, a touch input device, a touch-screen device, an interactive display device, a mouse, and/or the like.

The bus 210 represents what may be one or more busses (such as, for example, an address bus, data bus, or combination thereof). Similarly, in embodiments, the computing device 200 may include a number of processors 220, a number of memory components 230, a number of I/O ports 240, a number of I/O components 250, and/or a number of power supplies 260. Additionally any number of these components, or combinations thereof, may be distributed and/or duplicated across a number of computing devices.

In embodiments, the memory 230 includes computer-readable media in the form of volatile and/or nonvolatile memory and may be removable, nonremovable, or a combination thereof. Media examples include Random Access Memory (RAM); Read Only Memory (ROM); Electronically Erasable Programmable Read Only Memory (EEPROM); flash memory; optical or holographic media; magnetic cassettes, magnetic tape, magnetic disk storage or other magnetic storage devices; data transmissions; and/or any other medium that can be used to store information and can be accessed by a computing device such as, for example, quantum state memory, and/or the like. In embodiments, the memory 230 stores computer-executable instructions 290 for causing the processor 220 to implement aspects of embodiments of system components discussed herein and/or to perform aspects of embodiments of methods and procedures discussed herein.

The computer-executable instructions 290 may include, for example, computer code, machine-useable instructions, and the like such as, for example, program components capable of being executed by one or more processors 220 associated with the computing device 200. Program components may be programmed using any number of different programming environments, including various languages, development kits, frameworks, and/or the like. Some or all of the functionality contemplated herein may also, or alternatively, be implemented in hardware and/or fi rmware.

The illustrative computing device 200 shown in FIG. 2 is not intended to suggest any limitation as to the scope of use or functionality of embodiments of the present disclosure. Neither should the illustrative computing device 200 be interpreted as having any dependency or requirement related to any single component or combination of components illustrated therein. Additionally, various components depicted in FIG. 2 may be, in embodiments, integrated with various ones of the other components depicted therein (and/or components not illustrated), all of which are considered to be within the ambit of the present disclosure.

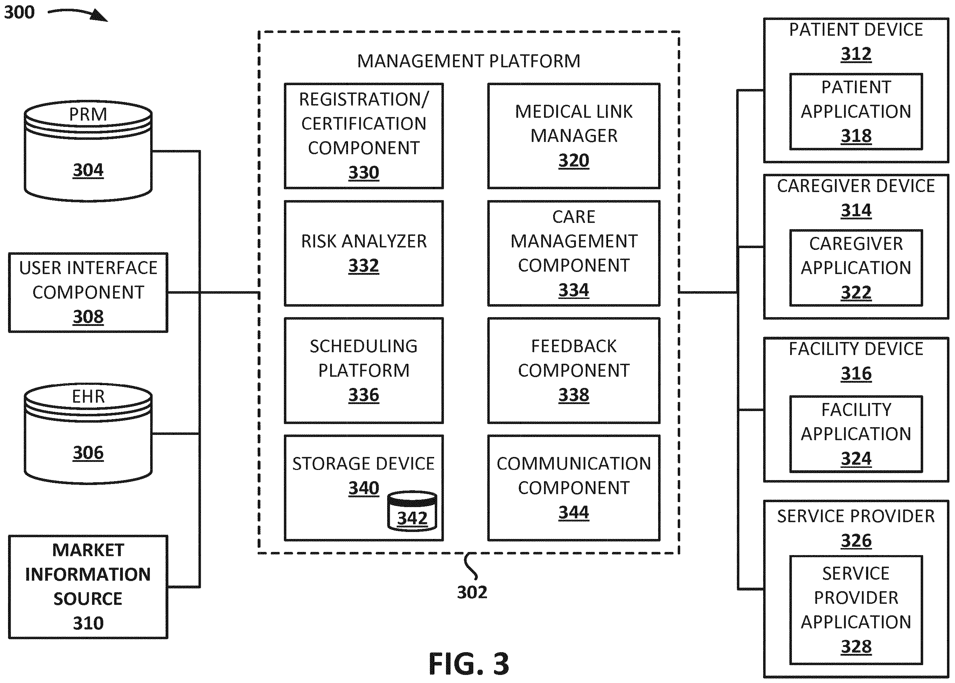

FIG. 3 is another block diagram depicting an illustrative health management system 300, which may be, include, or be included in, a clinical resource management system, in accordance with embodiments of the disclosure. As shown, the system 300 includes a management platform 302 (e.g., the management platform 102 depicted in FIG. 1) that is communicably coupled to information sources such as, e.g., a PRM database 304, an EHR database 306, a user interface component 308, and a market information source 310. The management platform 302 is configured to receive patient information from the information sources 304, 306, 308, and 310 (any one or more of which may be examples of the information source 106 depicted in FIG. 1).

In embodiments, the PRM database 304 may include any number of different types of information associated with a patient (generally information that is at least partially different than the clinical information available from the EHR database 306). For example, the PRM database 304 may include at least one of psychosocial information, experiential information, relational information, preferential information, demographic information, barrier information, and compliance information. In embodiments, the PRM database 304 includes, incorporates, or is coupled to modules that enable the gathering of PRM information. These information gathering modules may include, for example, software programs, data entry forms, and/or the like. According to embodiments, a remote patient monitoring module may be used to gather information such as, for example, by providing an interactive experience on a mobile device, obtaining information from a medical device, and/or the like.

An example of a data gathering module includes a barrier assessment module. A barrier assessment module may be used to gather information associated with barriers to treatment, improvement, recovery, etc. that a particular patient faces. In embodiments, the results of the barrier assessment module may be used as part of a process of selecting a caregiver to satisfy a particular expert request. A barrier assessment may be designed, for example, to identify challenges that exist for patients and their caregivers that make it difficult for the patient to maintain good health and/or that otherwise contribute to a patient's risk score. Barriers may include behavior (e.g., an unwillingness to see doctors, etc.); mental state (e.g., depression, a lack of trust for caregivers, etc.); family issues (e.g., divorce, responsibilities associated with caring for an ill family member, etc.); medication/procedure side-effects; financial situation (e.g., uninsured, underinsured, inability to pay for care services, etc.); education (e.g., lack of general education, lack of specific education related to the patient's health, etc.); language; culture (e.g., religious beliefs, cultural norms, etc.); and/or the like. A barrier assessment module may include a questionnaire that elicits answers that are used to identify patient risk factors. A patient satisfaction module may be used, in embodiments, to gather patient satisfaction information which may be used to identify risk factors such as a patient's confidence in a care team, a patient's willingness to engage with a care team, or a patient's activation level. According to embodiments, a barrier assessment may be performed by a patient or by a healthcare provider with a patient, and may be administered, for example, via a website, in person, on paper, on a computer, on a mobile device, and/or the like.

The EHR database 306 may be provided by an EHR system, integrated with the management platform 302, and/or the like. The EHR database 306 may include any number of different types of clinical information associated with any number of different patients. The clinical information may be provided to the database 306 using any number of different types of electronic medical record (EMR) reporting architectures, PHR systems, direct entry, and/or the like.

The management platform 302 also is communicably coupled to one or more access devices such as, for example, a patient device 312, a caregiver device 314, and a facility device 316 (any one or more of which may be examples of the access device 112 depicted in FIG. 1). According to embodiments, the patient device 312 may be configured to instantiate a medical link application 318 (referred to herein as a "patient application") that is configured to interact with a medical link manager 320 implemented on the management platform 302. For example, the medical link manager 320 may be, or include, a server, and the patient application 318 may be, or include, a client application such that the medical link manager 320 and the patient application 318 interact using to client-server communication techniques. In embodiments, the patient application 318 may be configured to provide, to the patient, caregiver information associated with caregivers that accept an expert request, and/or caregivers that are selected to satisfy the expert request. The patient application 318 may be configured to facilitate interaction with a facility administrator, another caregiver, and/or the like (e.g., by facilitating communication with another medical link application to enable the patient to work with the other person to select a caregiver to satisfy an expert request. In embodiments, the patient application 318 also may be configured to facilitate evaluation of caregivers, expertise domains, and/or the like.

Similarly, the caregiver device 314 may instantiate a medical link app 322 (referred to herein as a "caregiver application"); and the facility device 316 may instantiate a medical link app 324 (referred to herein as a "facility application"). The caregiver application 322 and the facility application 324 may be similar to the patient application 318, and may also be configured to interact with the medical link manager 320. In embodiments, for example, the caregiver application 322 may be configured to facilitate registering a caregiver account, provide certification information to the medical link manager 320, receive notifications of expert requests, provide user interfaces for reviewing expert requests and responding to them, provide user interfaces that facilitate evaluating caregivers, and/or the like. In embodiments, for example, the facility application 324 may be configured to facilitate registration of a facility account, provide facility information to the medical link manager 320, provide expert requests to the medical link manager 320, provide user interfaces for reviewing caregiver information associated with caregivers that accept expert requests, receive user selections of caregivers, etc., provide user interfaces that facilitate evaluating caregivers, and/or the like.

The medical link manager 320 may be, be similar to, include, or be included in the medical link manager 108 depicted in FIG. 1. According to embodiments, the medical link manager 320 may be configured to receive (e.g., from a facility, the risk analyzer 332, a patient, another caregiver, etc.) an expert request associated with an expertise domain, the expertise domain corresponding to at least one of a product and a procedure. The expert request may include patient information, a proposed procedure date, an indication of a product, an indication of a procedure type, an estimated cost, and/or the like. The medical link manager 320 may be further configured to identify a caregiver that is certified as an expert with regard to the expertise domain; and provide, to a caregiver device associated with the caregiver, a notification of the expert request. In embodiments, the medical link manager 320 may be configured to receive, from the caregiver device, an acceptance indication indicating that the caregiver has accepted the expert request. As explained above, the medical link manager 320 may receive, from at least one additional caregiver device, at least one additional acceptance indication indicating that at least one additional caregiver has accepted the expert request. In response to selecting, or receiving an indication of a user selection of, a caregiver, the medical link manager 320 may be configured to provide, to the caregiver device associated with the selected caregiver, an acceptance confirmation indicating that the caregiver's acceptance satisfies the expert request.

The medical link manager 320 may be configured to use various algorithms and mathematical modeling such as, for example, trend and statistical analysis, data mining, pattern recognition, cluster analysis, neural networks and fuzzy logic. The medical link manager 320 may perform deterministic and probabilistic calculations. Deterministic calculations include algorithms for which a correlation is ascertained between the data analyzed and a given outcome. In addition, patient-specific clinical information may be stored and tracked for hundreds of thousands of individual patients, enabling a first-level electronic clinical analysis of the patient's clinical status and an intelligent estimate of the patient's short-term clinical prognosis, as well as a prediction of expertise domains that are likely to be implicated. The medical link manager 320 may be capable of selecting and/or suggesting caregivers with increasing levels of sophistication by taking into consideration a number of interacting factors, all of which may serve individually or collectively to select an appropriate caregiver.

The management platform 302 may also be communicably coupled to a service provider 326 (e.g., the service provider 110 depicted in FIG. 1). The service provider 326 may instantiate a medical link app 328 (referred to herein as a "service provider application"). In embodiments, the service provider application 328 may be, or provide a service such as, for example, by providing a website through which users may access, or otherwise interact with, the management platform 302. In embodiments, the service provider application 328 may be similar to the patient application 318, and may also be configured to interact with the medical link manager 320. The management platform 302 may be configured to provide notifications, caregiver information, care metrics, risk scores and/or other information to the access devices 312, 314, and 316 and the service provider 326, and/or to receive information from the access devices 312, 314, and 316 and the service provider 326 and the service provider 316. In embodiments, a service provider may be, include, or be included within, a component of the management platform 302 in lieu of, or in addition to, the illustrated service provider 326. In embodiments, for example, the communication component 334, described below, may be used as a service provider.

The management platform 302 includes a registration/certification component 330, a risk analyzer 332, a care management component 334, a scheduling platform 336, and a feedback component 338. In embodiments, the components 320, 330, 332, 334, 336, and 338 may be implemented in any combination of hardware, software, and/or firmware, and may be implemented, at least in part, by a controller, a processor, and/or the like (not shown). The management platform 302 may include any number of other components or combination of components including, for example, a security component, a user authorization component, a software provisioning component, and/or the like.

The registration/certification component 330 may be configured to create and/or manage caregiver records, certify caregivers with respect to expertise domains, access and/or index information in a database (e.g., the database 342, discussed in further detail below), and/or the like. According to embodiments, for example, the registration/certification component 330 may be configured to create a caregiver record corresponding to a caregiver; and store, in the caregiver record, caregiver information corresponding to the caregiver. Caregiver information may include any number of different types of information including, for example, demographic information associated with the caregiver, performance information associated with the caregiver; and/or the like. In embodiments, the registration/certification component 330 may be configured to receive certification information associated with the caregiver and corresponding to an expertise domain; and certify, based on the certification information, the caregiver as an expert with regard to the expertise domain.

In embodiments, the registration/certification component 330 is configured to certify the caregiver as an expert with regard to the expertise domain by: determining that the caregiver satisfies a set of expert criteria associated with the expertise domain; storing a certification indication that indicates that the caregiver is an expert with regard to the expertise domain; and associating the certification indication with the expertise domain and the caregiver record. For example, certification information may include an indication of an amount of training completed with respect to the expertise domain. In this example, for instance, the registration/certification component 330 may determine that the caregiver satisfies a set of expert criteria associated with the expertise domain by determining that the caregiver has completed an amount of training with respect to the expertise domain that exceeds a specified threshold and/or falls within a specified range. In embodiments, certification information may include information associated with a license or certification issued by a board, government, or other similar authority. In embodiments, certification information may include information reflecting a level of skill, an amount of experience, and/or the like. For example, in embodiments, the registration/certification component 330 may be configured to administer an exam to determine whether the caregiver has a specified level of understanding.

In embodiments, the risk analyzer 332 may utilize patient information received from the information sources 304 and 306, queries (and/or other user input) received from the user interface component 308, and/or information from other relevant sources, to analyze information related to a patient, and provide predictive assessments of the patient's well-being. These predictive assessments may include risk scores. In embodiments, for example, the risk score may include a score corresponding to at least one of a risk of admission (e.g., to a hospital or other clinical setting), a risk of readmission (e.g., to a hospital or other clinical setting), a risk of hospital utilization (which may, e.g., include outpatient and/or emergency services), a risk of high care cost (e.g., defined with reference to a threshold established by a user, a machine-learning algorithm, etc.), a risk of injury (e.g., a risk of falling), a risk of decompensation (e.g., a risk of mental, emotional, and/or physical health deterioration due to an existing illness or condition), a risk of noncompliance (e.g., a risk of noncompliance with a medication prescription, an exercise regimen, etc.), a risk of exacerbation of the patient's illness and/or condition, a risk of an adverse event (e.g., a risk of an undesirable health event or episode, a risk of an accident resulting from an undesirable health event or episode, etc.), and/or the like. The risk analyzer 332 also may identify one or more risk factors that contribute to the risk score, a level of contribution of each of the factors, and/or the like. Risk factors may include any number of different types of factors such as, for example, presence of an illness; stage of an illness; historical treatment outcomes; certain types of comorbidity (e.g., heart failure with comorbid COPD); age; sex; activity level; family history; address (e.g., location of residence); discharge day (e.g., day of the week, date, etc.); number of hospital admissions; rate of hospital admissions (e.g., number of hospital admissions with a specified time period); date of last hospital admission; diet; ward or unit of hospital in which the patient is placed or from which the patient has been discharged; family (e.g., whether the patient is married, has children, etc.); patient satisfaction with caregivers; level of patient education; language barriers; and/or the like.

According to embodiments, the risk analyzer 332 may be configured to facilitate clinical resource management by generating expert requests, providing analytical services regarding product and/or procedure markets, and/or the like. In embodiments, for example, the risk analyzer 332 may be configured to receive a set of patient information associated with a patient; and determine, based on the set of patient information, a risk score indicating a likelihood that the patient may benefit from a treatment corresponding to the expertise domain. In embodiments, the risk score may be, or represent, a probability that a patient or patients will develop a condition or illness for which a treatment corresponds to the expertise domain. The risk score may be a combination of a number of risk scores associated with individual patients. The risk analyzer 332 may be configured to compare the risk score to a specified threshold or range. If the risk score exceeds the specified threshold (or fails to reach the specified threshold, depending upon the configuration of the threshold), falls within the specified range (or falls outside of the specified range), the risk analyzer 332 may, in response, generate an expert request.

According to embodiments, the risk analyzer 332 may compare the risk score to risk scores associated with other expertise domains and, in embodiments, the risk analyzer 332 may assign and/or access weights associated with expertise domains, which may be used to facilitate prioritization of expert requests. For example, the risk analyzer 332 might determine a first risk score corresponding to a first expertise domain and a first facility, and a second risk score corresponding to a second expertise domain and a second facility. The risk analyzer 332 may compare the first risk score to the second risk score and, in embodiments, generate an expert request corresponding to the expertise domain that is associated with the higher (or lower) risk score. The risk score might apply a weight to each risk score based on the expertise domain. For example, each expertise domain may be assigned a weight based on its importance, risk level, urgency, and/or the like. That is, for example, though the first risk score may be greater than the second risk score, the first expertise domain may include a weight that, when combined with the first risk score, causes the weighted first risk score to be lower than the weighted second risk score, causing the risk analyzer 332 to generate an expert request corresponding to the second expertise domain and second facility. For example, the first expertise domain might correspond to an implantable cardiac monitor, while the second expertise domain might correspond to a procedure for implanting a pacemaker. In this example, the second expertise domain might be weighted more heavily than the first due to the significance of the potential risks associated with the second expertise domain, as compared to the relative potential risks associated with the first expertise domain. In this manner, the risk analyzer 332 may be used to facilitate focusing clinical resource management efforts in facilities and/or regions in which the efforts may produce more advantages than they might in other facilities and/or regions.

In embodiments, the care management component 334 may be configured to manage health care of patients, including creating and managing care pathways, analyzing patient information in the context of care pathways (e.g., to assess care pathway compliance), identify care episodes, extract care episode information from patient information, index information using a database 342, and/or the like. According to embodiments, a user (e.g., a clinician or other care provider) may interact with the care management component 334 to develop a care pathway definition, modify care pathways, access historical care information, and/or the like.

The care management component 334 may also be configured to determine one or more care metrics based on patient information, one or more care pathway definitions, and/or the like. In embodiments, the care pathway manager 324 analyzes the information received from the various information sources. For example, the care pathway manager 324 may be configured to analyze historical symptoms, diagnoses, and outcomes along with time development of the diseases and co-morbidities. The scheduling platform 336 may be configured to facilitate putting the patient into contact with a care provider, schedule an appointment with a care provider, prioritize delivery of care services (e.g., based on care pathway definitions), and/or the like. For example, in embodiments, the scheduling platform 336 may be configured to create an appointment corresponding to an expert request; and provide, to the caregiver device associated with a selected caregiver, an appointment confirmation associated with the appointment.

The feedback component 338 may be configured to facilitate evaluation of caregivers, expertise domains, and/or the like. For example, in embodiments, the feedback component 338 may be configured to collect and/or analyze information associated with the performance, quality, usability, comfort, and/or effectiveness of a certain product, class of products, manufacturer, procedure, type of procedure, caregiver, facility, and/or the like. In embodiments, for example, the feedback component 338 may be configured to provide, to at least one of a facility device and a patient device, an evaluation user interface, the evaluation user interface including input mechanisms for receiving evaluation information. The feedback component 338 may be configured to receive, via the evaluation user interface, an evaluation of the selected caregiver, the evaluation including evaluation information; and associate, with the caregiver record, at least one of the evaluation information and information derived from the evaluation information. In embodiments, for example, information derived from evaluation information may include a quality score. A quality score may represent a combination of ratings associated with a number of different factors associated with a caregiver's performance, abilities, and/or the like. The evaluation information and/or the information derived therefrom (e.g., quality score) may be taken into consideration (e.g., by the medical link manager 320) when selecting, or suggesting selection of, a caregiver.

In embodiments, any one or more of the medical link manager 320, the registration/certification component 330, the risk analyzer 332, the care management component 334, the patient referral component 336, and the feedback component 336 may be configured to access a storage device 340 that may include a database 342. The database 342 may be, be similar to, include, or be included within the database 114 depicted in FIG. 1. For example, the database 342 may include a number of databases such as, for example, a patient database, a population database, a medical database, a general database, and/or the like. The database 342 may include patient specific data, including care pathway definitions, care episode codes, care episode information, care metrics, risk scores, risk factors, care team identifications, and/or the like. According to embodiments, the database 342 may include non-patient specific data, such as data relating to other patients and population trends. The database 342 may include epidemic-class device statistics, patient statistics, data relating to staffing by health care providers, environmental data, pharmaceuticals, and/or the like. Embodiments of the database 342 may include clinical data relating to the treatment of diseases, historical trend data for multiple patients in the form of a record of progression of their disease(s) along with markers of key events, and/or the like. The database 342 may include non-medical data related to the patient. In embodiments, the database 342 may include external medical records maintained by a third party, such as drug prescription records maintained by a pharmacy, providing information regarding the type of drugs that have been prescribed for a patient.

One or more of the components 320, 330, 332, 334, 336, 338, 340, and 342 in FIG. 3 may be configured to use various algorithms and mathematical modeling such as, for example, trend and statistical analysis, data mining, pattern recognition, cluster analysis, neural networks and fuzzy logic. For example, the risk analyzer 332 may perform deterministic and probabilistic calculations. In embodiments, the medical link manager 320, the registration/certification component 330, the risk analyzer 332, the care management component 334, the scheduling platform 336, and/or the feedback component 338 may include machine-learning capabilities. For example, the one or more of the components 320, 330, 332, 334, 336, and 338 may be implemented via a neural network (or equivalent) system. One or more of the components 320, 330, 332, 334, 336, and 338 may be partially trained or untrained. In embodiments, one or more of the components 320, 330, 332, 334, 336, and 338 may continue to learn and adjust as the advanced patient management system functions (i.e., in real time), or may remain at a given level of learning and only advanced to a higher level of understanding when manually allowed to do so.

In embodiments, the management platform 302 includes a communication component 344 that may be configured to facilitate interactions between aspects of the management platform 302 and access devices 312, 314, 316, service provider devices 326, and/or the like. For example, in response to instructions from another component (e.g., the medical link manager 320), the communication component 344 may manage medical devices, facilitate client-server interactions between medical link client applications (e.g., the patient application 318, the caregiver application 322, the facility application 324, the service provider application 328) and the medical link server (e.g., which may be, include, or be included within the medical link manager 320), perform diagnostic data recovery, program devices, and/or otherwise collect and/or deliver information as needed. In embodiments, the communication component 344 can manage a web interface that can be accessed by patients and/or caregivers. The information gathered by an implanted device may be periodically transmitted to a web site that is securely accessible to the caregiver and/or patient in a timely manner (e.g., via a caregiver portal). In embodiments, a patient accesses detailed health information with diagnostic recommendations based upon analysis algorithms derived from leading health care institutions.

The illustrative health management system 300 shown in FIG. 3 is not intended to suggest any limitation as to the scope of use or functionality of embodiments of the present disclosure. Neither should the illustrative health management system 300 be interpreted as having any dependency or requirement related to any single component or combination of components illustrated therein. Additionally, various components depicted in FIG. 3 may be, in embodiments, integrated with various ones of the other components depicted therein (and/or components not illustrated), all of which are considered to be within the ambit of the present disclosure. For example, any one or more of the components 320, 330, 332, 334, 336, 338, and 344 may be integrated with any one or more of the other components 320, 330, 332, 334, 336, 338, and 344.

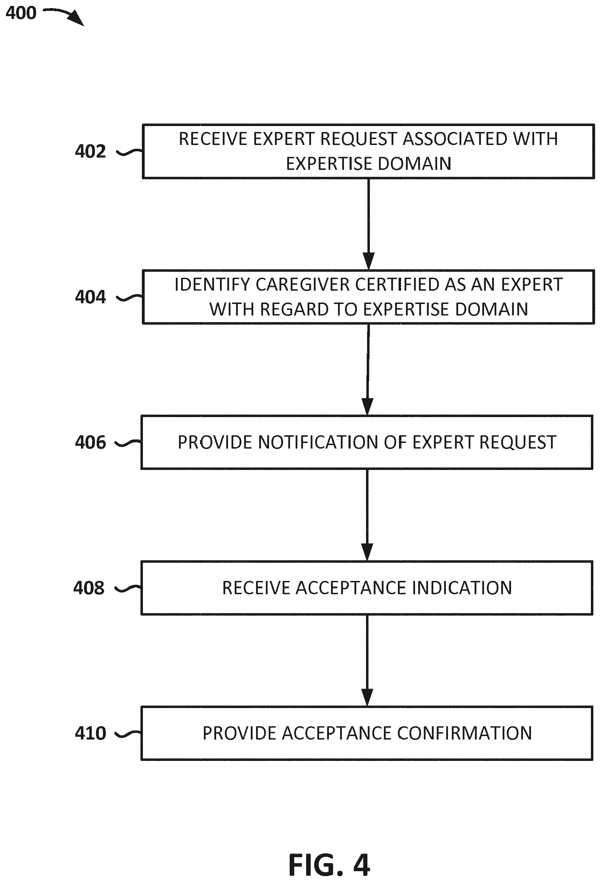

According to embodiments, systems discussed herein may facilitate clinical resource management. FIG. 4 is a flow diagram depicting an illustrative method 400 of clinical resource management, in accordance with embodiments of the disclosure. Aspects of embodiments of the illustrative method 400 may be performed by any number of different components discussed above with regard to FIGS. 1 - 3. As shown in FIG. 4, embodiments of the method 400 include receiving an expert request associated with an expertise domain (block 402). As described above, the expertise domain may correspond to a product and/or a procedure. Embodiments of the method 400 may include receiving an expert request from a facility device, a facility application, and/or the like. In embodiments, the expert request may include patient information, a proposed procedure date, an indication of a product, an indication of a procedure type, an estimated cost, and/or the like.

The method 400 also may include identifying a caregiver that is certified as an expert with regard to the expertise domain (block 404) and providing, to a caregiver device associated with the caregiver, a notification of the expert request (block 406). Embodiments of the method 400 further include receiving, from the caregiver device, an acceptance indication indicating that the caregiver has accepted the expert request (block 408). Embodiments of the method 400 may include any number of other steps not illustrated in FIG. 4. For example, embodiments of the method 400 may further include receiving, from at least one additional caregiver device, at least one additional acceptance indication indicating that at least one additional caregiver has accepted the expert request. In such a situation, embodiments of the method 400 may further include displaying, via a facility application, a representation of the caregiver and at least one additional representation of the at least one additional caregiver. The representations of the caregivers may include selectable options such that, when a user (e.g., a facility administrator, a patient, etc.) selects a selectable option corresponding to a caregiver, caregiver information is presented. In this manner, any number of different types of information may be provided to facilitate selection of a caregiver with respect to a particular expert request. The method 400 may also include providing, to the caregiver device associated with the selected caregiver, an acceptance confirmation indicating that the caregiver's acceptance satisfies the expert request (block 410).

In embodiments, the method 400 may further include receiving (e.g., from a facility application, a patient application, etc.) an indication of a user selection of a selected caregiver; and scheduling, via a scheduling platform, an appointment corresponding to the expert request. In response to scheduling the appointment, embodiments may include providing, to the caregiver device associated with the selected caregiver, an appointment confirmation associated with the appointment. Further, in embodiments, the method 400 may include receiving, from at least one of the facility device and a patient device, an evaluation of the selected caregiver, the evaluation comprising evaluation information; and associating, with the caregiver record, at least one of the evaluation information and information derived from the evaluation information, the information derived from the evaluation information comprising a quality score.

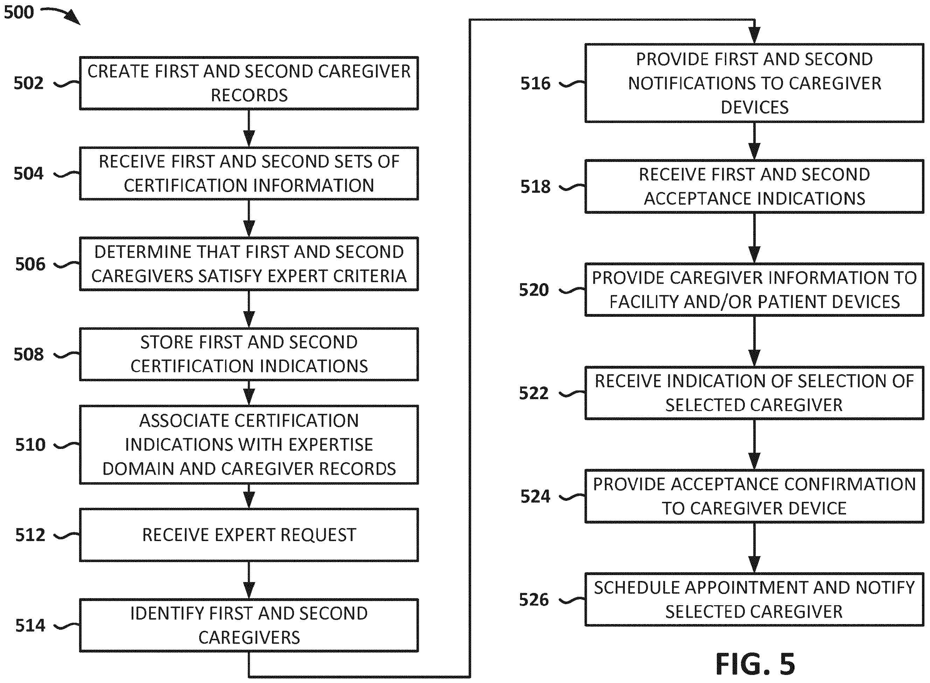

FIG. 5 is a flow diagram depicting another illustrative method 500 of clinical resource management, in accordance with embodiments of the disclosure. Aspects of embodiments of the illustrative method 500 may be performed by any number of different components discussed above with regard to FIGS. 1 - 3. Although the illustrative method 500 is described in the context of two caregivers, it will be understood by those having skill in the relevant arts, that embodiments of the method 500 may be implemented with respect to any number of caregivers.

As shown, embodiments of the method 500 include creating, in a database stored in computer memory, a first caregiver record and a second caregiver record, the first and second caregiver records corresponding to a first caregiver and a second caregiver, respectively (block 502). The illustrative method 500 further includes receiving a first set of certification information and a second set of certification information, the first set of certification information and the second set of certification information corresponding to the first caregiver and the second caregiver, respectively (block 504). Based on the first and second sets of certification information, respectively, a determination is made (e.g., by the registration/certification component 330 depicted in FIG. 3) that the first caregiver and second caregiver satisfy a first set of expert criteria associated with an expertise domain, the expertise domain corresponding to at least one of a product and a procedure (block 506). In embodiments, for example, determining that the first caregiver satisfies a first set of expert criteria associated with an expertise domain may include determining that the first caregiver has completed a specified level of training associated with the expertise domain.

As shown in FIG. 5, the method 500 further includes storing a first certification indication and a second certification indication, the first and second certification indications indicating that the first and second caregivers, respectively, are experts with regard to the expertise domain (block 508); and associating the first and second certification indications with the expertise domain and the first and second caregiver records, respectively (block 510). Embodiments of the method 500 further include receiving an expert request associated with the expertise domain (block 512) and identifying, based on the first and second certification indications, the first and second caregivers, respectively (block 514). Notifications of the expert request are provided to a first caregiver device associated with the first caregiver and a second caregiver device associated with the second caregiver (block 516). Embodiments of the method 500 also include receiving, from the first and second caregiver devices, a first acceptance indication and a second acceptance indication, respectively, the first and second acceptance indications respectively indicating that the first and second caregivers have accepted the expert request (block 518).

As shown in FIG. 5, embodiments of the method 500 include providing, to at least one of a facility device and a patient device, caregiver information associated with the first and second caregivers (block 520); and receiving an indication of a selection of a selected caregiver (e.g., the first caregiver) (block 522). In embodiments, receiving an indication of a selection of a selected caregiver may include automatically selecting the caregiver and receiving an indication of that automatic selection. In embodiments, an indication of selection of the selected caregiver may be received from another device and/or program component such as, for example, from a facility application and/or a patient application. The method 500 also includes creating, via a scheduling platform, an appointment corresponding to the expert request (block 524); and providing, to the caregiver device associated with the selected caregiver, an appointment confirmation associated with the appointment (block 526). According to embodiments, the method 500 may include any number of other steps such as, for example, receiving, from at least one of the facility device and a patient device, an evaluation of the selected caregiver, the evaluation comprising evaluation information; and associating, with the first caregiver record, at least one of the evaluation information and information derived from the evaluation information, the information derived from the evaluation information comprising a quality score.

Various modifications and additions can be made to the exemplary embodiments discussed without departing from the scope of the present disclosure. For example, while the embodiments described above refer to particular features, the scope of this disclosure also includes embodiments having different combinations of features and embodiments that do not include all of the described features. Accordingly, the scope of the present disclosure is intended to embrace all such alternatives, modifications, and variations as fall within the scope of the claims, together with all equivalents thereof.

The application is directed, inter alia, at the following aspects:
1. A method of clinical resource management, the method comprising:
   receiving an expert request associated with an expertise domain, the expert request comprising at least one of patient information, a proposed procedure date, an indication of a product, an indication of a procedure type, and an estimated cost;
   identifying a caregiver, wherein the caregiver is certified as an expert with regard to the expertise domain;
   providing, to a caregiver device associated with the caregiver, a notification of the expert request;
   receiving, from the caregiver device, an acceptance indication indicating that the caregiver has accepted the expert request; and
   providing, to the caregiver device associated with the selected caregiver, an acceptance confirmation indicating that the caregiver's acceptance satisfies the expert request.
2. The method of aspect 1, the expertise domain corresponding to at least one of a product and a procedure.
3. The method of aspect 1, further comprising:
   storing, in a caregiver record, caregiver information corresponding to the caregiver;
   receiving certification information associated with the caregiver, the certification information corresponding to an expertise domain; and
   certifying the caregiver as an expert with regard to the expertise domain.
4. The method of aspect 1, wherein certifying the caregiver as an expert with regard to the expertise domain, comprises:
   determining that the caregiver satisfies a set of expert criteria associated with the expertise domain;
   storing a certification indication that indicates that the caregiver is an expert with regard to the expertise domain; and
   associating the certification indication with the expertise domain and the caregiver record.
5. The method of aspect 1, further comprising receiving, from at least one additional caregiver device, at least one additional acceptance indication indicating that at least one additional caregiver has accepted the expert request.
6. The method of aspect 5, further comprising displaying, via an access device, a representation of the caregiver and at least one additional representation of the at least one additional caregiver.
7. The method of aspect 6, further comprising receiving, from the access device, an indication of a user selection of a selected caregiver, the selected caregiver comprising one of the caregiver and the at least one additional caregiver.
8. The method of any of aspects 1-7, further comprising:
   creating, via a scheduling platform, an appointment corresponding to the expert request; and
   providing, to the caregiver device associated with the selected caregiver, an appointment confirmation associated with the appointment.
9. The method of any of aspects 1-8, further comprising:
   receiving an evaluation of the selected caregiver, the evaluation comprising evaluation information; and
   associating, with the caregiver record, at least one of the evaluation information and information derived from the evaluation information, the information derived from the evaluation information comprising a quality score.
10. A health management system, the system comprising:
   at least one processor; and
   one or more computer-readable media having computer-executable instructions embodied thereon that, when executed by the at least one processor, cause the at least one processor to instantiate at least one program component, the at least one program component comprising a medical link manager configured to:
      receive an expert request associated with an expertise domain, the expertise domain corresponding to at least one of a product and a procedure, the expert request comprising at least one of patient information, a proposed procedure date, an indication of a product, an indication of a procedure type, and an estimated cost;
      identify a caregiver, wherein the caregiver is certified as an expert with regard to the expertise domain;
      provide, to a caregiver device associated with the caregiver, a notification of the expert request;
      receive, from the caregiver device, an acceptance indication indicating that the caregiver has accepted the expert request; and
      provide, to the caregiver device associated with the selected caregiver, an acceptance confirmation indicating that the caregiver's acceptance satisfies the expert request.
11.The system of aspect 10, the at least one program component further comprising a registration/certification component configured to:
   store, in a caregiver record, caregiver information corresponding to the caregiver;
   receive certification information associated with the caregiver, the certification information corresponding to an expertise domain; and
   certify the caregiver as an expert with regard to the expertise domain, wherein the registration/certification component is configured to certify the caregiver as an expert with regard to the expertise domain by:
      determining that the caregiver satisfies a set of expert criteria associated with the expertise domain;
      storing a certification indication that indicates that the caregiver is an expert with regard to the expertise domain; and
      associating the certification indication with the expertise domain and the caregiver record.
12. The system of either of aspects 10 or 11, wherein the medical link manager is further configured to:
   receive, from at least one additional caregiver device, at least one additional acceptance indication indicating that at least one additional caregiver has accepted the expert request; and
   receive an indication of a user selection of a selected caregiver, the selected caregiver comprising one of the caregiver and the at least one additional caregiver.
13. The system of any of aspects 10-12, further comprising a scheduling platform configured to:
   create an appointment corresponding to the expert request; and
   provide, to the caregiver device associated with the selected caregiver, an appointment confirmation associated with the appointment.
14. The system of any of aspects 10-12, further comprising a feedback component configured to:
   provide, to at least one of a facility device and a patient device, an evaluation user interface, the evaluation user interface comprising input mechanisms for receiving evaluation information;
   receive, via the evaluation user interface, an evaluation of the selected caregiver, the evaluation comprising evaluation information; and
   associate, with the caregiver record, at least one of the evaluation information and information derived from the evaluation information, the information derived from the evaluation information comprising a quality score.
15. A method of clinical resource management, the method comprising:
   creating, in a database stored in computer memory, a first caregiver record and a second caregiver record, the first and second caregiver records corresponding to a first caregiver and a second caregiver, respectively;
   receiving a first set of certification information and a second set of certification information, the first set of certification information and the second set of certification information corresponding to the first caregiver and the second caregiver, respectively;
   determining, based on the first set of certification information, that the first caregiver satisfies a first set of expert criteria associated with an expertise domain, the expertise domain corresponding to at least one of a product and a procedure;
   storing a first certification indication, the first certification indication indicating that the first caregiver is an expert with regard to the expertise domain;
   associating the first certification indication with the expertise domain and the first caregiver record;
   determining, based on the second set of certification information, that the second caregiver satisfies a set of expert criteria associated with the expertise domain;
   storing a second certification indication, the second certification indication indicating that the second caregiver is an expert with regard to the expertise domain;
   associating the second certification indication with the expertise domain and the second caregiver record;
   receiving an expert request associated with the expertise domain;
   identifying, based on the first and second certification indications, the first and second caregivers, respectively;
   providing, to a first caregiver device associated with the first caregiver, a first notification of the expert request;
   providing, to a second caregiver device associated with the second caregiver, a second notification of the expert request;
   receiving, from the first and second caregiver devices, a first acceptance indication and a second acceptance indication, respectively, the first and second acceptance indications respectively indicating that the first and second caregivers have accepted the expert request;
   providing, to an access device, a representation of each of the first and second caregivers;
   receiving, from the access device, an indication of a selection of a selected caregiver, the selected caregiver comprising the first caregiver; and
   providing, to the first caregiver device, an acceptance confirmation indicating that the caregiver's acceptance satisfies the expert request.
16. A method of clinical resource management, the method comprising:
   receiving an expert request associated with an expertise domain, the expert request comprising at least one of patient information, a proposed procedure date, an indication of a product, an indication of a procedure type, and an estimated cost;
   identifying a caregiver, wherein the caregiver is certified as an expert with regard to the expertise domain;
   providing, to a caregiver device associated with the caregiver, a notification of the expert request;
   receiving, from the caregiver device, an acceptance indication indicating that the caregiver has accepted the expert request; and
   providing, to the caregiver device associated with the selected caregiver, an acceptance confirmation indicating that the caregiver's acceptance satisfies the expert request.
17. The method of aspect 16, the expertise domain corresponding to at least one of a product and a procedure.
18. The method of aspect 16, further comprising:
   storing, in a caregiver record, caregiver information corresponding to the caregiver;
   receiving certification information associated with the caregiver, the certification information corresponding to an expertise domain; and
   certifying the caregiver as an expert with regard to the expertise domain.
19. The method of aspect 16, wherein certifying the caregiver as an expert with regard to the expertise domain, comprises:
   determining that the caregiver satisfies a set of expert criteria associated with the expertise domain;
   storing a certification indication that indicates that the caregiver is an expert with regard to the expertise domain; and
   associating the certification indication with the expertise domain and the caregiver record.
20. The method of aspect 16, further comprising receiving, from at least one additional caregiver device, at least one additional acceptance indication indicating that at least one additional caregiver has accepted the expert request.
21. The method of aspect 20, further comprising:
   displaying, via a user interface, a representation of the caregiver and at least one additional representation of the at least one additional caregiver; and
   receiving, via the user interface, an indication of a user selection of a selected caregiver, the selected caregiver comprising one of the caregiver and the at least one additional caregiver.
22. The method of aspect 20, further comprising automatically selecting a caregiver, the selected caregiver comprising one of the caregiver and the at least one additional caregiver.
23. The method of aspect 16, further comprising:
   creating, via a scheduling platform, an appointment corresponding to the expert request; and
   providing, to the caregiver device associated with the selected caregiver, an appointment confirmation associated with the appointment.
24. The method of aspect 16, further comprising:
   receiving, from at least one of the facility device and a patient device, an evaluation of the selected caregiver, the evaluation comprising evaluation information; and
   associating, with the caregiver record, at least one of the evaluation information and information derived from the evaluation information, the information derived from the evaluation information comprising a quality score.
25. A health management system, the system comprising:
   at least one processor; and
   one or more computer-readable media having computer-executable instructions embodied thereon that, when executed by the at least one processor, cause the at least one processor to instantiate at least one program component, the at least one program component comprising a medical link manager configured to:
      receive an expert request associated with an expertise domain, the expert request comprising at least one of patient information, a proposed procedure date, an indication of a product, an indication of a procedure type, and an estimated cost;
      identify a caregiver, wherein the caregiver is certified as an expert with regard to the expertise domain;
      provide, to a caregiver device associated with the caregiver, a notification of the expert request;
      receive, from the caregiver device, an acceptance indication indicating that the caregiver has accepted the expert request; and
      provide, to the caregiver device associated with the selected caregiver, an acceptance confirmation indicating that the caregiver's acceptance satisfies the expert request.
26. The system of aspect 25, the expertise domain corresponding to at least one of a product and a procedure.
27. The system of aspect 25, the at least one program component further comprising a registration/certification component configured to:
   store, in a caregiver record, caregiver information corresponding to the caregiver;
   receive certification information associated with the caregiver, the certification information corresponding to an expertise domain; and
   certify the caregiver as an expert with regard to the expertise domain, wherein certifying the caregiver as an expert with regard to the expertise domain, comprises:
      determining that the caregiver satisfies a set of expert criteria associated with the expertise domain;
      storing a certification indication that indicates that the caregiver is an expert with regard to the expertise domain; and
      associating the certification indication with the expertise domain and the caregiver record.
28. The system of aspect 25, wherein the medical link manager is further configured to:
   receive, from at least one additional caregiver device, at least one additional acceptance indication indicating that at least one additional caregiver has accepted the expert request; and
   receive an indication of a user selection of a selected caregiver, the selected caregiver comprising one of the caregiver and the at least one additional caregiver.
29. The system of aspect 25, further comprising a scheduling platform configured to:
   create an appointment corresponding to the expert request; and
   provide, to the caregiver device associated with the selected caregiver, an appointment confirmation associated with the appointment.
30. The system of aspect 25, further comprising a risk analyzer configured to:
   receive a set of patient information associated with a patient;
   determine, based on the set of patient information, a risk score indicating a likelihood that the patient may benefit from a treatment corresponding to the expertise domain;
   determine that the risk score exceeds a specified threshold; and
   generate, in response to determining that the risk score exceeds the specified threshold, the expert request.
31. A method of clinical resource management, the method comprising:
   creating, in a database stored in computer memory, a first caregiver record and a second caregiver record, the first and second caregiver records corresponding to a first caregiver and a second caregiver, respectively;
   receiving a first set of certification information and a second set of certification information, the first set of certification information and the second set of certification information corresponding to the first caregiver and the second caregiver, respectively;
   determining, based on the first set of certification information, that the first caregiver satisfies a first set of expert criteria associated with an expertise domain, the expertise domain corresponding to at least one of a product and a procedure;
   storing a first certification indication, the first certification indication indicating that the first caregiver is an expert with regard to the expertise domain;
   associating the first certification indication with the expertise domain and the first caregiver record;
   determining, based on the second set of certification information, that the second caregiver satisfies a set of expert criteria associated with the expertise domain;
   storing a second certification indication, the second certification indication indicating that the second caregiver is an expert with regard to the expertise domain;
   associating the second certification indication with the expertise domain and the second caregiver record;
   receiving an expert request associated with the expertise domain;
   identifying, based on the first and second certification indications, the first and second caregivers, respectively;
   providing, to a first caregiver device associated with the first caregiver, a first notification of the expert request;
   providing, to a second caregiver device associated with the second caregiver, a second notification of the expert request;
   receiving, from the first and second caregiver devices, a first acceptance indication and a second acceptance indication, respectively, the first and second acceptance indications respectively indicating that the first and second caregivers have accepted the expert request;
   providing, to at least one of a facility device and a patient device, caregiver information associated with the first and second caregivers;
   receiving, from the facility device, an indication of a selection of a selected caregiver, the selected caregiver comprising the first caregiver; and
   providing, to the first caregiver device, an acceptance confirmation indicating that the caregiver's acceptance satisfies the expert request.
32. The method of aspect 31, further comprising:
   creating, via a scheduling platform, an appointment corresponding to the expert request; and
   providing, to the caregiver device associated with the selected caregiver, an appointment confirmation associated with the appointment.
33. The method of aspect 31, further comprising:
   receiving, from at least one of the facility device and a patient device, an evaluation of the selected caregiver, the evaluation comprising evaluation information; and
   associating, with the first caregiver record, at least one of the evaluation information and information derived from the evaluation information, the information derived from the evaluation information comprising a quality score.
34. The method of aspect 33, wherein the caregiver information associated with the first caregiver includes a prior quality score.
35. The method of aspect 31, wherein determining that the first caregiver satisfies a first set of expert criteria associated with an expertise domain comprises determining that the first caregiver has completed a specified level of training associated with the expertise domain.

## Claims

1. A method of clinical resource management, the method comprising:
receiving an expert request associated with an expertise domain, the expert request comprising at least one of patient information, a proposed procedure date, an indication of a product, an indication of a procedure type, and an estimated cost;
identifying a caregiver, wherein the caregiver is certified as an expert with regard to the expertise domain;
providing, to a caregiver device associated with the caregiver, a notification of the expert request;
receiving, from the caregiver device, an acceptance indication indicating that the caregiver has accepted the expert request; and
providing, to the caregiver device associated with the selected caregiver, an acceptance confirmation indicating that the caregiver's acceptance satisfies the expert request.

2. The method of claim 1, the expertise domain corresponding to at least one of a product and a procedure.

3. The method of claim 1, further comprising:
storing, in a caregiver record, caregiver information corresponding to the caregiver;
receiving certification information associated with the caregiver, the certification information corresponding to an expertise domain; and
certifying the caregiver as an expert with regard to the expertise domain.

4. The method of claim 1, wherein certifying the caregiver as an expert with regard to the expertise domain, comprises:
determining that the caregiver satisfies a set of expert criteria associated with the expertise domain;
storing a certification indication that indicates that the caregiver is an expert with regard to the expertise domain; and
associating the certification indication with the expertise domain and the caregiver record.

5. The method of claim 1, further comprising receiving, from at least one additional caregiver device, at least one additional acceptance indication indicating that at least one additional caregiver has accepted the expert request.

6. The method of claim 5, further comprising displaying, via an access device, a representation of the caregiver and at least one additional representation of the at least one additional caregiver.

7. The method of claim 6, further comprising receiving, from the access device, an indication of a user selection of a selected caregiver, the selected caregiver comprising one of the caregiver and the at least one additional caregiver.

8. The method of any of claims 1-7, further comprising:
creating, via a scheduling platform, an appointment corresponding to the expert request; and
providing, to the caregiver device associated with the selected caregiver, an appointment confirmation associated with the appointment.

9. The method of any of claims 1-8, further comprising:
receiving an evaluation of the selected caregiver, the evaluation comprising evaluation information; and
associating, with the caregiver record, at least one of the evaluation information and information derived from the evaluation information, the information derived from the evaluation information comprising a quality score.

10. A health management system, the system comprising:
at least one processor; and
one or more computer-readable media having computer-executable instructions embodied thereon that, when executed by the at least one processor, cause the at least one processor to instantiate at least one program component, the at least one program component comprising a medical link manager configured to:
receive an expert request associated with an expertise domain, the expertise domain corresponding to at least one of a product and a procedure, the expert request comprising at least one of patient information, a proposed procedure date, an indication of a product, an indication of a procedure type, and an estimated cost;
identify a caregiver, wherein the caregiver is certified as an expert with regard to the expertise domain;
provide, to a caregiver device associated with the caregiver, a notification of the expert request;
receive, from the caregiver device, an acceptance indication indicating that the caregiver has accepted the expert request; and
provide, to the caregiver device associated with the selected caregiver, an acceptance confirmation indicating that the caregiver's acceptance satisfies the expert request.

11. The system of claim 10, the at least one program component further comprising a registration/certification component configured to:
store, in a caregiver record, caregiver information corresponding to the caregiver;
receive certification information associated with the caregiver, the certification information corresponding to an expertise domain; and
certify the caregiver as an expert with regard to the expertise domain, wherein the registration/certification component is configured to certify the caregiver as an expert with regard to the expertise domain by:
determining that the caregiver satisfies a set of expert criteria associated with the expertise domain;
storing a certification indication that indicates that the caregiver is an expert with regard to the expertise domain; and
associating the certification indication with the expertise domain and the caregiver record.

12. The system of either of claims 10 or 11, wherein the medical link manager is further configured to:
receive, from at least one additional caregiver device, at least one additional acceptance indication indicating that at least one additional caregiver has accepted the expert request; and
receive an indication of a user selection of a selected caregiver, the selected caregiver comprising one of the caregiver and the at least one additional caregiver.

13. The system of any of claims 10-12, further comprising a scheduling platform configured to:
create an appointment corresponding to the expert request; and
provide, to the caregiver device associated with the selected caregiver, an appointment confirmation associated with the appointment.

14. The system of any of claims 10-12, further comprising a feedback component configured to:
provide, to at least one of a facility device and a patient device, an evaluation user interface, the evaluation user interface comprising input mechanisms for receiving evaluation information;
receive, via the evaluation user interface, an evaluation of the selected caregiver, the evaluation comprising evaluation information; and
associate, with the caregiver record, at least one of the evaluation information and information derived from the evaluation information, the information derived from the evaluation information comprising a quality score.

15. A method of clinical resource management, the method comprising:
creating, in a database stored in computer memory, a first caregiver record and a second caregiver record, the first and second caregiver records corresponding to a first caregiver and a second caregiver, respectively;
receiving a first set of certification information and a second set of certification information, the first set of certification information and the second set of certification information corresponding to the first caregiver and the second caregiver, respectively;
determining, based on the first set of certification information, that the first caregiver satisfies a first set of expert criteria associated with an expertise domain, the expertise domain corresponding to at least one of a product and a procedure;
storing a first certification indication, the first certification indication indicating that the first caregiver is an expert with regard to the expertise domain;
associating the first certification indication with the expertise domain and the first caregiver record;
determining, based on the second set of certification information, that the second caregiver satisfies a set of expert criteria associated with the expertise domain;
storing a second certification indication, the second certification indication indicating that the second caregiver is an expert with regard to the expertise domain;
associating the second certification indication with the expertise domain and the second caregiver record;
receiving an expert request associated with the expertise domain;
identifying, based on the first and second certification indications, the first and second caregivers, respectively;
providing, to a first caregiver device associated with the first caregiver, a first notification of the expert request;
providing, to a second caregiver device associated with the second caregiver, a second notification of the expert request;
receiving, from the first and second caregiver devices, a first acceptance indication and a second acceptance indication, respectively, the first and second acceptance indications respectively indicating that the first and second caregivers have accepted the expert request;
providing, to an access device, a representation of each of the first and second caregivers;
receiving, from the access device, an indication of a selection of a selected caregiver, the selected caregiver comprising the first caregiver; and
providing, to the first caregiver device, an acceptance confirmation indicating that the caregiver's acceptance satisfies the expert request.
